Europäisches Patentamt

European Patent Office

Office européen des brevets

(11) Veröffentlichungsnummer: **0 216 994**
**B1**

(12) **EUROPÄISCHE PATENTSCHRIFT**

(45) Veröffentlichungstag der Patentschrift: **16.05.90**

(51) Int. Cl.⁵: **G 01 N 11/14**

(21) Anmeldenummer: **86107219.7**

(22) Anmeldetag: **28.05.86**

(54) Verfahren zur Bestimmung der Gelierzeit von Reaktionsharzen.

(30) Priorität: **07.06.85 AT 1707/85**

(43) Veröffentlichungstag der Anmeldung:
**08.04.87 Patentblatt 87/15**

(45) Bekanntmachung des Hinweises auf die
Patenterteilung:
**16.05.90 Patentblatt 90/20**

(84) Benannte Vertragsstaaten:
**AT BE CH DE FR GB IT LI NL SE**

(56) Entgegenhaltungen:
**DE-A-1 648 833**
**GB-A-1 561 323**

**Prospekt "reiter", Reiter Kunstharzprodukte
GmbH T1 6002, GELTIMER 1,
GELIERUNGSZEIT-MESSGERÄT**
**Prospekt "reiter", Reiter Kunstharzprodukte
GmbH T1 6003, GELTIMER 2, GELIERUNGSZEIT-
MESSGERÄT**

(73) Patentinhaber: **Vianova Kunstharz
Aktiengesellschaft**
**A-8402 Werndorf (AT)**

(72) Erfinder: **Gessner, Werner**
**Auersperggasse 23**
**A-8010 Graz (AT)**
Erfinder: **Wetz, Gerhard**
**Koloniegasse 25**
**A-8020 Graz (AT)**

Courier Press, Leamington Spa, England.

**Beschreibung**

Die Erfindung betrifft ein verbessertes Verfahren zur Bestimmung der Gelierzeit von Reaktionsharzen, insbesonders von ungesättigten Polyesterharzen (UP-Harzen), wie sie vom Deutschen Normenausschuß in der DIN 16 945 (Ausgabe April 1976) beschrieben ist.

Durch die Gelierzeit wird normgemäß ein Anhalt dafür gegeben, wann und bei welcher Temperatur die Reaktionsharzmasse nach Zugabe von Härter und Beschleuniger in den Gelzustand übergeht. (Die Temperatur (=Prüftemperatur) wird üblicherweise vom Leiferanten des Harzes angegeben). In diesem Normblatt werden zwei Verfahren zur Bestimmung der Glierzeit angegeben.

Bei dem im Normblatt angegebene "Verfahren A" wird die Gelierzeit durch regelmäßiges händischen Heben und Senken eines Glasstabs in der in einem Reagenzglas befindlichen Rekationsharzmasse bestimmt. Der Endpunkt ist dann gegeben, wenn beim Anheben des Glasstabs das Reagenzglas infolge der Gelierung des Harzes mitgehoben wird.

Beim "Verfahren B" wird der Glasstab über eine Magnetkupplung und einen Stahldraht an die Welle eines Motors angeschlossen. Beim Eintreten der Gelierung wird der Glasstab abgebremst. Dadurch wird eine Vorrichtung (z.B. Kontaktstreifen oder elektronische Einrichtungen) ausgelöst, welche den Motor und die Zeitmesseung stoppen. Für dieses "Verfahren B" wurden von der Industrie verschiedene Geräte entwickelt, welche die Änderung des Widerstandes bei der Gelierung und die entsprechende Zeit in verschiedenster Weise anzeigen (siehe z.B. Merkblätter über "GELTIMER" der Fa. REITER, Wien).

Bei beiden Verfahren ist für das Signal "Ende der Meßzeit" das Auftreten einer bestimmten Kraft bzw. eines bestimmten Drehmomentes erforderlich, wobei die ursprüngliche Viskosität des zu messenden Reaktionsharzes unberückschichtigt bleibt. In der Praxis bedeutet das, daß bei dünnflüssigen Produkten das Signal zu spät kommt. Bei zähflüssigen Mischungen wird das Signal zu früh bzw. sofort nach dem Eintauchen des Prüfkörpers erfolgen. Es ist offensichtlich, daß die Meßergebnisse daher in vielen Fällen fragwürdig oder überhaupt unbrauchbar sind.

Die angegebenen Meßmethoden werden vor allem auch dann problematisch, wenn bei einem Verarbeiter von Reaktionsharzen oder bei einem Hersteller solcher Produkte eine breite Palette verschiedener Harztypen mit unterschiedlicher Viskosität geprüft werden muß. Besondere Schwierigkeiten treten dabei immer dann auf, wenn Harze mit längerer Gelierperiode bzw. mit nichtnewtonschem Fließverhalten (z.B. thixotropierte Harzkompositionen) geprüft werden, da einerseits beim Eintreten der Gelierung noch für längere Zeit keine Anzeige erfolgt, andererseits das Harz zum Zeitpunkt der Anzeige längst nicht mehr verarbeitungsfähig ist. In diesen Fällen bringt beim heutigen Stand der Technik nur die Handmethode (Verfahren "A") ein praxisgerechtes Ergebnis, da durch die händische Bedienung auch eine Beobachtung des Harzzustandes möglich ist und der Gelierungsbeginn exakt festgehalten werden kann. Beim Anfall einer größeren Anzahl von Messungen ergibt sich daraus jedoch ein erheblicher Arbeitsaufwand.

In der DE—OS—16 48 833 wird eine Vorrichtung zum Messen der Erstarrung fließfähiger Massen beschrieben, bei welcher ein zylinderförmiger Körper in der erstarrenden Masse rotiert. Durch die Änderung des Drehmoments kann der Verlauf des Erstarrungsvorganges ermittelt werden. Die Vorrichtung entspricht in ihrer Wirkungsweise im wesenltichen den Prüfgeräten gemäß DIN 16 945, Verfahren B.

Das verwendete Meßsystem besteht aus einer feststehenden, geteilten, an den Wänden gerieften, zylindrischen Probenkammer und einem sich drehenden, gerieften Zylinder. Es wird für die rheologische Untersuchung von sehr hochviskosen Preßmassen eingesetzt. Dazu sind sehr große Drehmomente erforderlich (daher Riefung der Wandlfächen). Die Momente, welche durch die Lagerreibung hervorgerufen werden, spielen eine untergeordnete Rolle.

Für die Messung von niedrig- bis mittelviskosen Substanzen wäre die in der DE—OS—16 48 833 aufgezeigte Anordnung sehr ungünstig, da die Substanz durch den Spalt des geteilten Gehäuses und durch die Lagerspalte austreten würde, außerdem werden die Lager vom Meßgut umströmt. Es treten sehr unterschiedliche Schergefälle auf, sodaß aufgrund des geforderten großen Viskositätsbereiches mit erheblichen Fehlern zu rechnen wäre. Zusätzlich ist ein erheblicher Aufwand für die Demontage und Reinigung des Meßsystems nach der Messung zu erwarten.

Das in der GB—PS—15 61 323 beschriebene Viskosimeter verwendet einen Rotationskörper, der eine im wesentlichen zylindrische Form besitzt, welche zur Erleichterung des Entweichens von Luftblasen beim vollständigen Eintauchen ins Meßgut und des Abfließens der Meßlösung am oberen und unteren Ende in einer schwach konischen Form ausgeführt ist. Da der Rotationskörper für die Messung vollständig in der Flüssigkeit eingetaucht ist, hat die Form des Rotationskörpers keine Meßtechnisch funktionelle Bedeutung.

Die Messung der Gelierzeit mit diesem Gerät wäre mit Schwierigkeiten verbunden, da die zirkulierende Strömung beim Einsetzen der Gelierung, also beim wesentlichen Teil des Meßvorganges, nicht aufrechterhalten werden kann.

Es wurde nun gefunden, daß durch eine verbesserte Meßanordnung, insbesonders bezüglich der Form und Eintauchtiefe des Rotationskörpers die Genauigkeit und Reproduzierbarkeit der Gelierzeitmessung bei Reaktionsharzen wesentlich verbessert werden kann.

Die vorliegende Erfindung betrifft demgemäß ein verbessertes Verfahren zur Bestimmung der Gelierzeit von Reaktionsharzen gemäß DIN 16 945, "Verfahren B", die eine Viskosität zwischen ca. 500 und ca. 50.000 mPa · s aufweisen, wobei der Verlauf der Erstarrung durch Messung der Änderung des Drehmomentes eines mit konstanter Drehzahl laufenden Motors (4), an dessen Welle ein Rotationskörper

2

(1) angebracht ist, der durch eine Absenkvorrichtung (2) an einem Stativ (3) in die frisch bereitete, vorzugsweise in einem Thermostatbad (7) befindliche Harz/Härter/Beschleuniger-Mischung (8) eingetaucht wird, verfolgt werden kann, welches dadurch gekennzeichnet ist, daß der in einer konischen Form ausgebildete Rotationskörper (1) so weit in die Harzmischung eingetaucht wird, bis ein die Stromaufnahme des Motors (4) anzeigendes oder überwachendes Gerät (5) einen für den jeweiligen Gerätetyp festgelegten Anfangswert, entsprechend einem Drehmoment M1, signalisiert, bei dieser viskositätsabhängigen Eintauchtiefe des Rotationskörpers der Geliervorgang abläuft und durch eine Zeitmeßvorrichtung (6), die bei Erreichen eines festgelegten, höherliegenden zweiten Drehmomentes M2 gestoppt wird, die Gelierzeit bestimmt wird.

Die Erfindung betrifft weiters eine in vorliegenden Anspruch 2 definierte Vorrichtung zur Durchführung des Verfahrens.

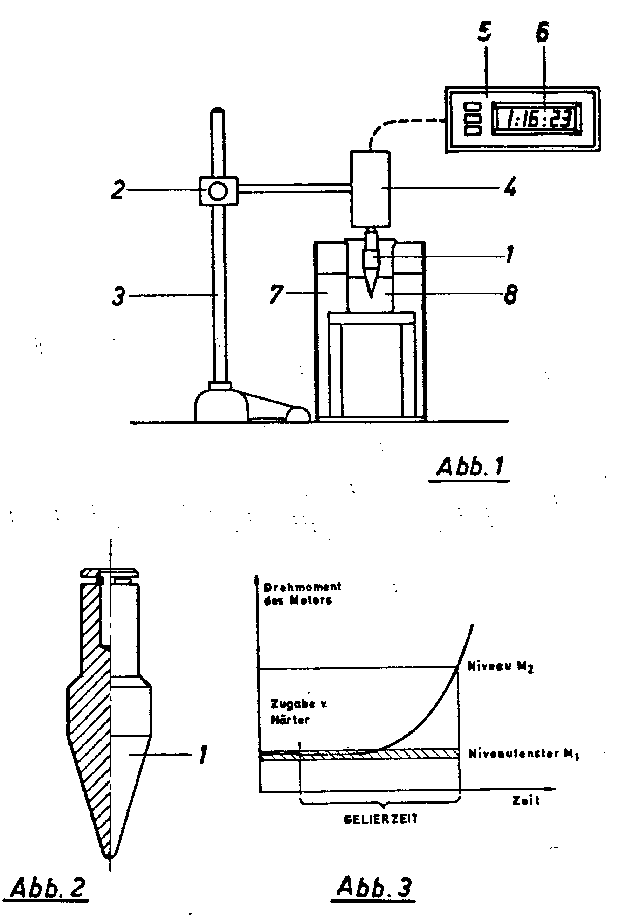

Eine Ausführungsform für diese Vorrichtung wird in der Abb. 1 dargestellt, wobei (1) einen konisch ausgebildeten Rotationskörper, (2) eine Vorrichtung zur Absenkung des aus dem Motor (4) und dem Rotationskörper (1) bestehenden Meßteiles an einem Stativ (3) in die in einem Thermostatbad (7) befindliche Harzmischung (8) bedeutet. (5) ist ein mit einer Zeitmeßvorrichtung (6) ausgestattetes und das Drehmoment anzeigendes Gerät.

Abb. 2 zeigt die wesentlichen Merkale des konischen Rotationskörpers. Abb. 3 gibt den typischen Verlauf des Drehmomentes bei der Härtung eines Rotationsharzes wieder.

Durch das erfindungsgemäße Verfahren wird die Gelierzeit durch ein Viskositätsverhältnis charakterisiert, welches in weiten Grenzen unabhängig vom absoluten Wert der Ausgangsviskosität ist. Aus der Beziehung

$$M = k \cdot \eta \cdot R^3,$$

wobei M das Moment an der Motowelle, k eine Apparatekonstante, $\eta$ die dynamische Viskosität und R der wirksame Radius des Rotationskörpers bedeutet, ist ersichtlich, daß mit dem erfindungsgemäßen Verfahren in einem weiten Viskositätsbereich gearbeitet werden kann.

Neben der Übereinstimmung der Meßergebnisse nach dem erfindungsgemäßen Verfahren mit den nach dem händischen Verfahren erhaltenen Werten (unter der Voraussetzung der Beobachtung des Gelierungsbildes) und der großen Meßbreite für Harze verschiedener Viskosität hat das erfindungsgemäße Verfahren gegenüber den bekannten Vorrichtungen nach "Verfahren B" überdies weitere Vorteile.

Durch den Wegfall von Torsionsdrähten und Fühlereinrichtungen, welche immer wieder ausgewechselt werden müssen sowie gegebenenfalls durch den Wegfall spezieller nicht wieder verwendbarer Probengefäße ergibt sich eine wesentliche Herabsetzung der Betriebskosten und eine Steigerung der Einsatzbereitschaft des Gerätes. Durch die konische Form des abnehmbaren Rotationskörpers und das Weiterlaufen des Antriebes nach Einsetzen der Gelierung wird sowohl die Entfernung des Rotationskörpers aus der Reaktionsmasse, als auch dessen Reinigung problemlös möglich.

Der Rotationskörper ist entsprechend Abb. 2 ausgeführt und abnehmbar an der Welle des Antriebsmotors befestigt. Der Winkel der Kegelerzeugenden von der Konusachse des Rotationskörpers beträgt zwischen 10 und 60 Grad, vorzugsweise zwischen 15 und 30 Grad. Es ist offensichtlich, daß ein zu kleiner Winkel zu großen Eintauchtiefen führt bzw. ein zu großer Winkel die Genauigkeit der Einstellung des Niveaus M1 negativ beeinflußt.

Unter Verwendung des erfindungsgemäßen Verfahrens können in einwandfreier Weise die Gelierzeiten von Reaktionsharzen gemessen werden, welche eine Viskosität zwischen etwa 500 und etwa 50.000 mPa · s aufweisen. In praktisch allen Fällen wird eine ausgezeichnete Übereinstimmung mit dem tatsächlichen Gelierverhalten beobachtet.

In der einfachsten Weise erfolgt die Messung der Gelierzeit nach dem erfindungsgemäßen Verfahren in der Weise, daß man das thermostatisierte Reaktionsharz mit dem Härter und gegebenenfalls Beschleuniger versetzt und zu diesem Zeitpunkt die Zeitmessung in Tätigkeit setzt. Die Probe wird vorteilhafterweise in einer größeren Menge in einem getrennten Gefäß hergestellt und erst nach dem gründlichen Vermischen in das Meßgefäß eingefüllt. Der Rest der Probe kann für andere Bestimmungen, wie den Temperaturverlauf bei der Härtung u.ä. verwendet werden. Nach dem Einfüllen des Gemisches in das Meßgefäß wird der Rotationskörper so weit in die Reaktionsmasse eingesenkt, bis das für das jeweilige Meßgerät durch Eichung festgelegte Ausgangsniveau M1 angezeigt wird. Dieses Ausgangsniveau wird vorteilhafterweise als Fensterbereich definiert, d.h. das Signal erlischt bei zu großer und zu geringer Eintauchtiefe. Bei Erreichen des Niveaus M2 wird die Zeitmessung abgeschaltet.

Zur Erhöhung der Meßsicherheit und zur Rationalisierung kann das Verfahren in vielfältiger Weise ergänzt werden.

Eine Bedienungserleichterung stellt z.B. eine optische oder akustische Zeichengebung beim Erreichen der Niveaus M1 bzw. M2 dar. Ebenso kann durch ein motorisches Absenken des Rotationskörpers auf das Ausgangsniveau M1 der Vorgang weiter automatisiert werden. Ein Herausheben des Rotationskörpers nach Erreichen des Niveaus M2 vermeidet ein Verkleben mit dem gelierenden Harz und erleichtert die Reinigung.

Selbstverständlich ist es auch möglich, mehrere Meßstellen mit einem gemeinsamen Steuer- und Anzeigeteil zu verbinden. Die Meß- und Regeltechnik bietet solche Steuer- und Anzeigeorgane in vielen

EP 0 216 994 B1

Ausführungsformen an. Auch das Prinzip der Verwendung des Antriebsmotors als Meßwandler für das Drehmoment gehört zum Stand der Technik und wird z.B. bei einigen Typen von Rotationsviskosimetern angewandt. Selbstverständlich können die Meßwerte bzw. der Meßverlauf auch graphisch festgehalten werden.

Da die Geräte mit unterschiedlichen Formen des Rotationskörpers ausgestattet und Antriebsmotoren mit Getrieben verschiedene Charakteristik aufweisen können, ist eine typenmäßige Festlegung der Niveaus M1 und M2 durch eine einmalige Versuchsreihe mit verschiedenen Typen von Reaktionsharzen notwendig. Als Vergleich wird vorteilhafterweise die händische Methode unter gleichzeitiger Beobachtung des Gelierungsbeginns herangezogen. Eine laufende Überprüfung der Funktionsgenauigkeit kann durch Messung einer nichtgelierenden Flüssigkeit entsprechender Viskosität und Markierung der Eintauchtiefe am Rotationskörper erfolgen.

Die nachfolgenden Vergleichsdaten sollen die Übereinstimmung der Gelierzeiten beim erfindungsgemäßen Verfahren mit dem Handverfahren unter Beweis stellen Die dabei benützte Anordnung weist folgende Kennwerte auf:

Motor: Fahlhaber Typ 330/217 mit Getriebe 3,06:1 (Fa. Faulhaber, Schönaich, BRD).

Spindel: polierter Edelstrahlrotationskörper gemäß Abb. 2, Konuswinkel 20° (gemäß Definition), maximaler Durchmesser 15 mm, maximale Eintauchtiefe 40 mm.

Niveaufenster M1: ca. $5 \cdot 10^{-2}$ N $\cdot$ cm.

Niveaufenster M2: ca. $20 \cdot 10^{-2}$ N $\cdot$ cm.

Gemessen wurden die Gelierzeiten von handelsüblichen ungesättigten Polyesterharzen, welche mit den vom Hersteller angegebenen Härter- und Beschleunigermengen versetzt wurden.

| Viskositäts-bereich der Harze | Produkt | Handmessung (1) (Min.:Sek) | erfgem. Verfahren |
|---|---|---|---|
| (a) Niedrigviskos | A | 10:00 | 10:18 |
| (unter 500 mPa · s) | B | 10:30 | 10:17 |
| | | | |
| (b) Mittelviskos | | | |
| (1000—2000 mPa · s) | C | 9:00 | 8:57 |
| | D | 14:00 | 13:53 |
| | E | 23:00 | 23:15 |
| | F | 41:30 | 41:52 |
| | G | 57:30 | 59:46 |
| | | | |
| (c) Hochviskos | | | |
| (5000—15.000 mPa · s) | H | 14:30 | 15:18 |
| | I | 20:15 | 19:58 |
| | K | 35:00 | 33:38 |

Alle Werte resultieren als Mittelwert aus 3 Messungen. (1) mit opt. Beobachtung des Gelierbeginns.

**Patentansprüche**

1. Verbessertes Verfahren zur Bestimmung der Gelierzeit von Reaktionsharzen gemäß DIN 16 945, "Verfahren B", die eine Viskosität zwischen ca. 500 und ca. 50.000 mPa · s aufweisen, wobei der Verlauf der Erstarrung durch Messung der Änderung des Drehmomentes eines mit konstanter Drehzahl laufenden Motors (4), an dessen Welle ein Rotationskörper (1) angebracht ist, der durch eine Absenkvorrichtung (2) an einem Stativ (3) in die frisch bereitete, vorzugsweise in einem Thermostatbad (7) befindliche Harz/Härter/Beschleuniger-Mischung (8) eingetaucht wird, verfolgt werden kann, dadurch gekennzeichnet, daß der in einer konischen Form ausgebildete Rotationskörper (1) so weit in die Harzmischung eingetaucht wird, bis ein die Stromaufnahme des Motors (4) anzeigendes oder überwachendes Gerät (5) einen für den jeweiligen Gerätetyp festgelegten Anfangswert, entsprechend einem Drehmoment M1, signalisiert, bei dieser viskositätsabhängigen Eintauchtiefe des Rotationskörpers der Geliervorgang abläuft und durch eine Zeitmeßvorrichtung (6), die bei Erreichen eines festgelegten, höherliegenden zweiten Drehmomentes M2 gestoppt wird, die Gelierzeit bestimmt wird.

2. Vorrichtung zur Bestimmung der Gelierzeit von Reaktionsharzen gemäß DIN 16 945, "Verfahren B", die eine Viskosität zwischen ca. 500 und ca. 50.000 mPa · s aufweisen, wobei der Verlauf der Erstarrung durch Messung der Änderung des Drehmomentes eines mit konstanter Drehzahl laufenden Motors (4), an dessen Welle ein Rotationskörper (1) angebracht ist, der durch eine Absenkvorrichtung (2) an einem Stativ (3) in die frisch bereitete, vorzugsweise in einem Thermostatbad (7) befindliche Harz/Härter/Beschleuniger-Mischung (8) eingetaucht wird, verfolgt werden kann, dadurch gekennzeichnet, daß der Rotationskörper (1) in einer konischen Form, wobei der Winkel zur Konusachse 10 bis 60 Grad, vorzugsweise 15 bis 30 Grad, beträgt, ausgebildet ist und eine Anzeigevorrichtung (5) für mindestens 2 Drehmomentniveaus und eine

4

# EP 0 216 994 B1

Zeitmeßvorrichtung (6) zur Bestimmung des Zeitintervalles zwischen den Drehmomentniveaus vorhanden ist.

**Revendications**

1. Procédé perfectionné pour la détermination du temps de gélification de résines de réaction selon la norme DIN 16 945, "Procédé B", qui présentent une viscosité comprise entre environ 500 et environ 50 000 mPa · s, le déroulement de la solidification pouvant être suivi en mesurant la variation du couple de rotation d'un moteur (4) tournant à une vitesse angulaire constante, et sur l'arbre duquel est calé un corps rotatif (1) qui est plongé par l'intermédiaire d'un dispositif d'abaissement (2), sur un statif (3), dans le mélange (8) résine/durcisseur/activateur, fraîchement préparé et situé de préférence dans un bain thermostatique (7), caractérisé par le fait que le corps rotatif (1) réalisé sous une forme conique est plongé, dans le mélange à base de résine, jusqu'à ce qu'un appareil (5), affichant ou surveillant la consommation de courant du moteur (4), signale une valeur initiale spécifiée pour le type d'appareil considéré, en concordance avec un couple de rotation M1; le processus de gélification s'opère en présence de cette profondeur d'immersion du corps rotatif, dépendante de la viscosité; et le temps de gélification est déterminé par l'intermédiaire d'un dispositif chronométreur (6) qui est mis à l'arrêt lorsqu'un second couple de rotation M2, supérieur et spécifié, est atteint.

2. Dispositif pour la détermination du temps de gélification de résines de réaction selon la norme DIN 16 945, "Procédé B", qui présentent une viscosité comprise entre environ 500 et environ 50 000 mPa · s, le déroulement de la solidification pouvant être suivi en mesurant la variation du couple de rotation d'un moteur (4) tournant à une vitesse angulaire constante, et sur l'arbre duquel est calé un corps rotatif (1) qui est plongé par l'intermédiaire d'un dispositif d'abaissement (2), sur un statif (3), dans le mélange (8) résine/durcisseur/activateur, fraîchement préparé et situé de préférence dans un bain thermostatique (7), caractérisé par le fait que le corps rotatif (1) est réalisé sous une forme conique, l'angle par rapport à l'axe du cône mesurant de 10 à 60 degrés, de préférence entre 15 et 30 degrés; et par la présence d'un dispositif d'affichage (5) pour au moins 2 niveaux de couples de rotation, ainsi que d'un dispositif chronométreur (6) pour déterminer l'intervalle de temps entre les niveaux de couples de rotation.

**Claims**

1. Improved method for determining the gelling time of reactive resins, according to DIN 16 945, "Method B", which have a viscosity of between about 500 and about 50,000 mPa · s, it being possible to monitor the progress of the solidification by measuring the change in torque of a motor (4) running at constant speed of rotation, to the shaft of which a rotation body (1) is fitted, which is immersed by a lowering device (2) on a stand (3) into the freshly prepared resin/curing agent/accelerator mixture (8) preferably located in a thermostat bath (7), characterized in that the rotation body (1) formed in a conical shape is immersed into the resin mixture until an instrument (5) indicating or monitoring the power consumption of the motor (4) signals an initial value, fixed for the particular type of instrument, corresponding to a torque M1, the gelling process takes place at this viscosity-dependent depth of immersion of the rotation body and the gelling time is determined by a timing device (6) which is stopped when a fixed, higher second torque M2 is reached.

2. Apparatus for determining the gelling time of reactive resins according to DIN 16 945, "Method B", which have a viscosity of between about 500 and about 50,000 mPa · s, it being possible for the progress of the solidification to be monitored by measuring the change in torque of a motor (4) running at constant speed of rotation, to the shaft of which a rotation (1) is fitted which is immersed by a lowering device (2) on a stand (3) into the freshly prepared resin/curing agent/accelerator mixture (8) preferably located in a thermostat bath (7), characterized in that the rotation body (1) is formed in a conical shape, the angle to the cone axis being 10 to 60 degrees, preferably 15 to 30 degrees, and an indicator device (5) for at least two torque levels and a timing device (6) for determining the time interval between the torque levels are provided.

5   6

1:16:23

2

4

3

1

7

8

*Abb.1*

*Abb.2*

Drehmoment
des Moters

Niveau M₂

Zugabe x
Härter

Niveaufenster M₁

Zeit

GELIERZEIT

*Abb.3*